# EUROPÄISCHE PATENTSCHRIFT

(11) **EP 1 827 202 B1**
(45) Veröffentlichungstag und Bekanntmachung des Hinweises auf die Patenterteilung: **23.03.2011**
(21) Anmeldenummer: 05786366.4
(22) Anmeldetag: 10.09.2005
(51) Int. Cl.: A61B 3/06

(54) **VERFAHREN ZUR DURCHFÜHRUNG EINES KONTRASTSEHTESTS**
METHOD FOR PERFORMING A CONTRAST VISION TEST
PROCEDE PERMETTANT D'EFFECTUER UN TEST DE CONTRASTE VISUEL

(30) Priorität: 19.11.2004 DE 102004055754
(43) Veröffentlichungstag der Anmeldung: 05.09.2007
(73) Patentinhaber: Carl Zeiss Vision GmbH, 73430 Aalen (DE)
(72) Erfinder: KRATZER, Timo, 73434 Aalen (DE); HOFMANN, Erich, 73430 Aalen (DE); MENDEL, Lars, 73430 Aalen (DE)
(74) Vertreter: Lorenz, Werner
(86) Internationale Anmeldenummer: PCT/EP2005/009735
(87) Internationale Veröffentlichungsnummer: WO 2006/056252

(56) Entgegenhaltungen:
- DE-A1- 3 009 049
- US-A- 5 500 699
- US-A- 5 589 897
- US-A1- 2003 174 284

## Beschreibung

Die Erfindung betrifft ein Verfahren zur Durchführung eines Kontrastsehtests bei einer Testperson zur Bestimmung einer Kontrastsensitivitätsfunktion. Die Erfindung betrifft ebenfalls ein Computerprogramm zur Durchführung des Verfahrens, sowie ein Computerprogrammprodukt und einen Computer zur Ausführung des Computerprogramms.

Bei Kontrastsehtests wird die Kontrastsehfähigkeit einer Testperson mit Hilfe einer Kontrastsensitivitätsfunktion ermittelt. Dies geschieht bei bisherigen Geräten und Messverfahren durch Messung von diskreten Visus- (d.h. Sehschärfe-) und Kontrastwerten. Als Messobjekte kommen hierbei wie beispielsweise aus der US 5,500,699 bekannt, Landoltringe, sowie Buchstaben in bestimmten Größen mit bestimmen diskreten Kontrastswerten zum Einsatz. Außerdem werden teilweise Sinusgitter mit einer bestimmten festen Frequenz d.h. Ortsfrequenz und einem bestimmten Kontrastwert eingesetzt.

Derartige Tests werden bisher nur für sehbehinderte Personen oder zur Diagnose krankhafter Veränderungen des Sehapparates eingesetzt. Die Auswertung erfolgt üblicherweise durch Erfassen der diskreten Punkte in einem Visus-Kontrast-Schema und durch einen visuellen Vergleich verschiedener erfasster Daten.

Auch bei normalfehlsichtigen Personen kann die individuelle Kontrastsensitivitätsfunktion über die Sehleistung bzw. über Probleme mit der Sehleistung Aufschluss geben. Für diese Gruppe von Personen sind die diskreten Abstufungen der bekannten Kontrastsehtests allerdings zu grob. Des weiteren decken die bisherigen Tests nicht den gesamten, für normalfehlsichtige Personen in Frage kommenden Kontrast- und Visusbereich ab. Bei den bisherigen Auswerteverfahren besteht zudem die Problematik, dass keine kontinuierlichen Daten digital erfasst werden, die eine automatische Weiterverarbeitung ermöglichen würden.

Darüber hinaus existiert bisher keine aussagekräftige Metrik, mit der die Kontrastsensitivitätsfunktion beschrieben werden kann.

In der DE 30 09 049 ist eine Vorrichtung zum Prüfen des Sehvermögens mit einem Mittel zum Vorführen eines visuellen Zieles bzw. Bildes für eine Versuchsperson beschrieben, wobei das Bild eine Anordnung von zwei Sets von Bereichen jeweils unterschiedlicher Luminanz mit alternierender Sequenz in einer koordinierten Richtung quer zur Anordnung umfasst, und wobei ein Steuerungsmittel durch die Versuchsperson zur Veränderung des Bildkontrastes einstellbar ist. Mindestens die Bereiche eines Sets besitzen eine sich zunehmend verändernde Luminanz gegenüber der Luminanz des anderen Sets in einer anderen koordinierten Richtung quer zur Anordnung. Das Steuerungsmittel ist durch die Versuchsposition zur Änderung der Geschwindigkeit bzw. des Ausmaßes der Luminanzänderung einstellbar.

Der vorliegenden Erfindung liegt die Aufgabe zugrunde, ein Verfahren der eingangs erwähnten Art zu schaffen, das die Nachteile des Standes der Technik vermeidet und insbesondere einen aussagekräftigen Kontrastsehtest zur Bestimmung einer Kontrastsensitivitätsfunktion auch bei normalfehlsichtigen Testpersonen zur Verfügung stellt.

Diese Aufgabe wird erfindungsgemäß durch ein Verfahren mit den Merkmalen von Anspruch 1 gelöst.

Das vorgeschlagene Verfahren zur Durchführung eines Kontrastsehtests bei einer Testperson zur Bestimmung einer Kontrastsensitivitätsfunktion basiert auf einem kontinuierlichen Si nusgitter, bei dem sich auf der einen Achse die Frequenz des Sinusgitters kontinuierlich ändert, d.h. es ändert sich der für die Testperson zu erkennende Visus bzw. die Sehschärfe oder der zu erkennende Sehwinkel, während auf der dazu senkrechten Achse der Kontrast des Sinusgitters kontinuierlich variiert wird. Für jede Testperson gestattet der erfindungsgemäße Kontrastsehtest, ihre individuelle Kontrastsensitivitätsfunktion festzustellen. Diese Funktion ist diejenige einhüllende Kurve, unterhalb der sie noch ein Gittermuster erkennen kann. Auch bei normalfehlsichtigen Testpersonen kann in vorteilhafter Weise die individuelle Kontrastsensitivitätsfunktion bestimmt werden, denn für diese Personengruppe ist die feine kontinuierliche Auflösung des Sinusgitters ideal. Der vorgeschlagene Kontrastsehtest kann den gesamten, für normalfehlsichtige Personen in Frage kommenden Kontrast- und Visusbereich abdecken.

Des weiteren wird ein Computerprogramm, ein Computerprogrammprodukt und ein Computer zur Ausführung des Computerprogramms vorgeschlagen, um das erfindungsgemäße Verfahren durchzuführen.

Weitere vorteilhafte Ausgestaltungen und Weiterbildungen der Erfindung ergeben sich aus den restlichen Unteransprüchen. Nachfolgend ist anhand der Zeichnung prinzipmäßig ein Ausführungsbeispiel der Erfindung dargestellt.

Dabei zeigen:
- Figur 1: eine Darstellung eines kontinuierlichen Sinusgit- ters zur Verwendung bei einem erfindungsgemäßen Verfahren;
- Figur 2: eine Darstellung des kontinuierlichen Sinusgitters gemäß Figur 1 mit einer markierten Kontrast- sensitivitätskurve; und
- Figur 3: ein Diagramm einer Metrik zur Bewertung von zwei Kontrastsensitivitätskurven.

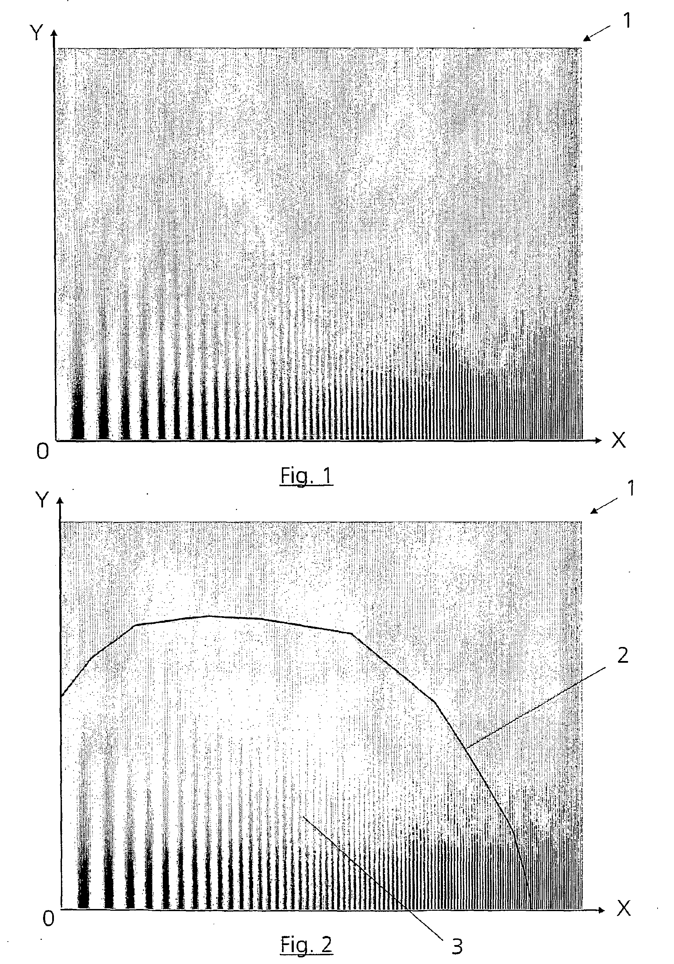

Bei einem Verfahren zur Durchführung eines Kontrastsehtests bei einer Testperson zur Bestimmung einer Kontrastsensitivitätsfunktion wird der Testperson eine Abbildung eines in Figur 1 dargestellten kontinuierlichen Sinusgitters 1 zur Betrachtung angezeigt, bei welchem sich die Ortsfrequenz kontinuierlich in Richtung einer X-Achse ändert, d.h. vorliegend ausgehend von einer Y-Achse erhöht und bei welchem sich der Kontrast auf der zu der X-Achse senkrechten Y-Achse kontinuierlich ändert, d.h. vorliegend ausgehend von der X-Achse verringert. Die Kontrastsensitivitätsfunktion der Testperson wird, wie aus Figur 2 ersichtlich, durch eine Kurve 2 definiert, die einen Bereich 3 des Sinusgitters 1 einschließt, in welchem die Testperson bei der Betrachtung das Sinusgittermuster noch wahrnehmen bzw. erkennen kann.

Die Ortsfrequenz- und/oder Kontrastbereiche des kontinuierlichen Sinusgitters 1 sind anpassbar. D.h. die Bereiche, die durch den Kontrastsehtest abgedeckt werden, können - sowohl in Visus- (X) als auch in Kontrastrichtung (Y) - den gewünschten Erfordernissen bzw. auf die Testpersonen angepasst werden.

Das Verfahren zur Durchführung eines Kontrastsehtests bei einer Testperson zur Bestimmung einer Kontrastsensitivitätsfunktion ist in vorteilhafter Weise als Computerprogramm auf einem Computer realisiert. In weiteren Ausführungsbeispielen kann das erfindungsgemäße Verfahren selbstverständlich auch mit anderen Hilfsmitteln durchgeführt werden. Durch die Abarbeitung auf einem Mikroprozessor des Computers wird das Computerprogramm ausgeführt. Das Computerprogramm kann auf einem computerlesbaren Datenträger (Diskette, CD, DVD, Festplatte, USB-Memorystick, oder dergleichen) oder einem Internet-Server als Computerprogrammprodukt gespeichert sein und von dort aus in einen Speicher des Computers übertragen werden. Die Abbildung des kontinuierlichen Sinusgitters 1 erfolgt auf einer Anzeigevorrichtung des Computers (nicht dargestellt). Der Computer bzw. die Anzeigevorrichtung, muss hierzu gewisse Voraussetzungen erfüllen. Die Auflösung der Anzeigevorrichtung muss so beschaffen sein, dass sie die fehlerfreie Wiedergabe des Maximalfrequenz des Sinusgitters 1 erlaubt. Um zweifelsfrei helle und dunkle Linien zu erhalten, muss auf jede helle bzw. dunkle Linie eine graue Linie folgen. Um dies zu erreichen, muss die Auflösung der Anzeigevorrichtung bzw. des Wiedergabegeräts mindestens 2 mal so groß sein, wie zur Darstellung des maximalen Visus erforderlich ist. Das Kontrastverhältnis der Anzeigevorrichtung muss mindestens so groß sein, dass die gewünschte Anzahl von verschiedenen Kontraststufen mit genügender Genauigkeit dargestellt werden kann.

Die Testperson markiert nun selbstständig die Kurve bzw. die Kontrastsensitivitätsfunktion 2 in dem Sinusgitter 1 mittels eines Eingabegeräts, insbesondere eines Zeigegeräts, bevorzugt einer Maus des Computers, wobei die markierte Kurve in dem Computer abgespeichert wird. D.h. der Proband kann seine individuelle Kontrastsensitivitätsfunktion mit der Maus oder einem anderen geeigneten Eingabegerät direkt eingeben (siehe Figur 2).

Wenn die Anzeigevorrichtung des Computers oder die Abbildung des kontinuierlichen Sinusgitters 1 auf der Anzeigevorrichtung des Computers um 90 Grad zur Hauptblickrichtung der Testperson schwenkbar bzw. drehbar ist, kann sowohl die horizontale Kontrastsensitivitätsfunktion, als auch die vertikale Kontrastsensitivitätsfunktion der Testperson erfasst werden.

Insbesondere kann über den Computer auch die minimale, die maximale und die mittlere Helligkeit des Sinusgitters 1 je nach Anforderung angepasst werden. Des weiteren kann die Grundfarbe auf die jeweiligen Erfordernisse angepasst werden. Vorliegend ist das Sinusgitter als Graustufengitter 1 ausgebildet. Denkbar sind beispielsweise auch Sinusgitter mit verschiedenen Rot-, Blau- oder Grüntönen, sowie weitere Farbvarianten.

Somit kann für jede Gebrauchs- bzw. Umgebungsbedingung (Umgebungshelligkeit, Grundhelligkeit des Kontrastsehtests, Blendung, verschiedene Sehhilfen oder dergleichen) ein Kontrastsehtest durchgeführt werden. Durch mehrfaches Durchführen des Kontrastsehtests bei vorgegebenen Umgebungsbedingungen und durch eine kontinuierliche Aufnahme von Daten, kann über statistische Methoden die individuelle Kontrastsensitivitätsfunktion der Testperson ermittelt werden. Durch Anbringen einer geeigneten Beleuchtung in der Nähe bzw. im Bereich der Anzeigevorrichtung kann der Kontrastsehtest auch unter Blendbedingungen durchgeführt werden.

Auf die Kontrastsensitivitätsfunktion können dann verschiedene Metriken angewandt werden, um die Kurve zu bewerten oder um verschiedene Kurven zu vergleichen. Geläufige Metriken sind beispielsweise der maximale Visus, der minimale Kontrast oder die Fläche unter der Kontrastsensitivitätskurve 2.

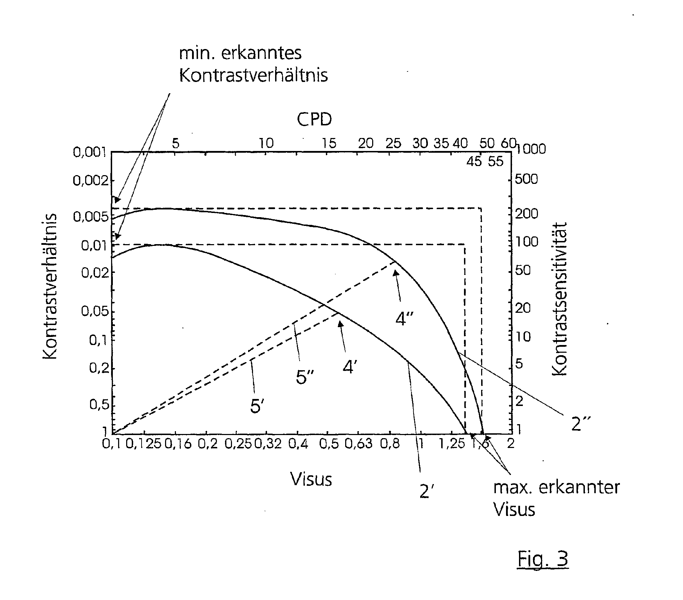

In Figur 3 wird als Metrik ein sogenanntes Contrast Acuity Produkt (CAP) verwendet. Dabei sind zwei Kontrastsensitivitätsfunktionen 2', 2 " vergleichend dargestellt. Im Diagramm sind Kontrastverhältnis (linke, vertikale Skala), Visus (untere, horizontale Skala), Kontrastsensitivität (rechte, vertikale Skala) und CPD, Cycles per Degree (obere, horizontale Skala) logarithmisch dargestellt. In die Berechnung des CAP gehen ein maximaler erkannter Visus, ein minimales erkanntes Kontrastverhältnis und eine Form bzw. eine Durchbiegung der Kontrastsensitivitätskurven 2', 2" multiplikativ ein. Die Form bzw. das Durchbiegen der Kurven 2', 2" wird durch die Schnittpunkte 4', 4" der jeweiligen Kontrastsensitivitätskurven 2', 2" mit der Diagonalen 5', 5" , die durch das Rechteck mit den Eckpunkten maximaler erkannter Visus, minimaler Visus (hier: 0,1), minimaler erkannter Kontrast, maximaler Kontrast (hier: 1,0) beschrieben wird, definiert.

Das CAP ist dann das Produkt der Werte maximaler erkannter Visus einer Kontrastsensitivitätskurve 2', 2", minimaler erkannter Kontrast einer Kontrastsensitivitätskurve 2', 2" und der Länge der Geraden 5', 5" von der linken unteren Ecke des dargestellten Bereiches zu dem oben beschriebenen Schnittpunkt 4', 4" einer Kontrastsensitivitätskurve 2', 2".

## Patentansprüche

1. Verfahren zur Durchführung eines Kontrastsehtests bei einer Testperson zur Bestimmung einer Kontrastsensitivitätsfunktion, wobei der Testperson eine Abbildung eines kontinuierlichen Sinusgitters (1) zur Betrachtung angezeigt wird, bei welchem sich die Ortsfrequenz kontinuierlich in Richtung einer ersten Achse (X) ändert und bei welchem sich der Kontrast in Richtung einer zu der ersten Achse (X) senkrechten zweiten Achse (Y) kontinuierlich ändert, wobei die Kontrastsensitivitätsfunktion der Testperson durch eine Kurve (2) definiert wird, die einen Bereich (3) des Sinusgitters (1) einschließt, in welchem die Testperson bei der Betrachtung das Sinusgittermuster wahrnimmt, **gekennzeichnet dadurch dass** erfasste Kurven (2', 2") mit einer geeigneten Metrik bewertet bzw. verglichen werden, wobei ein maximaler erkannter Visus, ein minimales erkanntes Kontrastverhältnis und eine Form der Kontrastsensitivitätskurve in die Bewertung mit einbezogen werden.

2. Verfahren nach Anspruch 1, **dadurch gekennzeichnet, dass** sich die Ortsfrequenz des Sinusgitters (1) ausgehend von der zweiten Achse (Y) kontinuierlich in Richtung der ersten Achse (X) erhöht und sich der Kontrast des Sinusgitters ausgehend von der ersten Achse (X) kontinuierlich in Richtung der zweiten Achse (Y) verringert.

3. Verfahren nach Anspruch 1 oder 2, **dadurch gekennzeichnet, dass** die Ortsfrequenz- und/oder Kontrastbereiche des kontinuierlichen Sinusgitters (1) anpassbar sind.

4. Verfahren nach Anspruch 1, 2 oder 3, **dadurch gekennzeichnet, dass** der Kontrastsehtest auf einem Computer durchgeführt wird und die Abbildung des kontinuierlichen Sinusgitters (1) auf einer Anzeigevorrichtung des Computers erfolgt.

5. Verfahren nach Anspruch 4, **dadurch gekennzeichnet, dass** die Abbildung des kontinuierlichen Sinusgitters (1) auf der Anzeigevorrichtung des Computers um 90 Grad zur Hauptblickrichtung der Testperson schwenkbar ausgebildet wird.

6. Verfahren nach einem der Ansprüche 1 bis 5, **dadurch gekennzeichnet, dass** der Kontrastsehtest mehrfach, insbesondere unter vorgegebenen Umgebungsbedingungen durchgeführt wird, wobei die Kontrastsensitivitätskurven aufgezeichnet werden.

7. Verfahren nach einem der Ansprüche 1 bis 6, **dadurch gekennzeichnet, dass** der Kontrastsehtest unter Blendbedingungen durchgeführt wird.

8. Verfahren nach einem der Ansprüche 4 bis 7, **dadurch gekennzeichnet, dass** die Testperson selbstständig die Kurve bzw. die Kontrastsensitivitätsfunktion mittels eines Eingabegeräts des Computers auf der Anzeigevorrichtung markiert, wobei die markierte Kurve (2) in dem Computer abgespeichert wird.

9. Computerprogramm mit Programmcodemitteln, um alle Schritte von jedem beliebigen der Ansprüche 1 bis 7 durchzuführen, wenn das Programm auf einem Computer ausgeführt wird.

10. Computerprogrammprodukt mit Programmcodemitteln, die auf einem computerlesbaren Datenträger gespeichert sind, um alle Schritte von jedem beliebigen der Ansprüche 1 bis 7 durchzuführen, wenn das Programmprodukt auf einem Computer ausgeführt wird.

11. Computerprogramm nach Anspruch 9 oder Computerprogrammprodukt nach Anspruch 10, **dadurch gekennzeichnet, dass** eine durch die Testperson mittels eines Eingabegeräts des Computers auf der Anzeigevorrichtung markierte Kurve in dem Computer abgespeichert wird.

12. Computer mit einer Anzeigevorrichtung und einem Eingabegerät, eingerichtet zur Ausführung des Computerprogramms gemäß Anspruch 9 oder 11.

13. Computer nach Anspruch 12, **dadurch gekennzeichnet, dass** die Anzeigevorrichtung um 90 Grad zur Hauptblickrichtung der Testperson schwenkbar ist.

14. Computer nach Anspruch 12 oder 13, **dadurch gekennzeichnet, dass** eine Beleuchtungseinrichtung im Bereich der Anzeigevorrichtung angeordnet ist.

## Claims

1. A method for performing a contrast vision test on a subject in order to determine a contrast sensitivity function, wherein an image of a continuous sinusoidal grating (1) is displayed for the subject to view, in which grating the spatial frequency continuously changes in the direction of a first axis (X) and in which the contrast continuously changes in the direction of a second axis (Y) which is perpendicular to the first axis (X), with the contrast sensitivity function of the subject being defined by a curve (2) which encloses an area (3) of the sinusoidal grating (1), in which the subject perceives the sinusoidal grating pattern during viewing, **characterized in that** registered curves (2, 2") are assessed or compared with a suitable metric, in which process the maximum identified visual acuity, the minimum identified contrast and a shape of the contrast sensitivity curve are included in the assessment.

2. The method as claimed in claim 1, **characterized in that** the spatial frequency of the sinusoidal grating (1) continuously increases in the direction of the first axis (X) starting from the second axis (Y) and the contrast of the sinusoidal grating continuously decreases in the direction of the second axis (Y) starting from the first axis (X).

3. The method as claimed in claim 1 or 2, **characterized in that** the spatial frequency and/or contrast ranges of the continuous sinusoidal grating (1) can be adapted.

4. The method as claimed in claim 1, 2 or 3, **characterized in that** the contrast vision test is performed on a computer and the continuous sinusoidal grating (1) is imaged on a display device of the computer.

5. The method as claimed in claim 4, **characterized in that** the image of the continuous sinusoidal grating (1) can be pivoted on the computer display device by 90 degrees with respect to the main viewing direction of the subject.

6. The method as claimed in one of claims 1 to 5, **characterized in that** the contrast vision test is repeated a number of times, in particular under the specified ambient conditions, in which process the contrast sensitivity curves are recorded.

7. The method as claimed in one of claims 1 to 6, **characterized in that** the contrast vision test is performed under glare conditions.

8. The method as claimed in one of claims 4 to 7, **characterized in that** the subject independently marks the curve or the contrast sensitivity function on the display device using an input appliance of the computer with the marked curve (2) being stored in the computer.

9. A computer program with program code means for performing all the steps of any of claims 1 to 7, if the program is executed on a computer.

10. A computer program product with program code means which are stored on a computer-readable data carrier for performing all the steps of any of claims 1 to 7, if the program product is executed on a computer.

11. The computer program as claimed in claim 9 or the computer program product as claimed in claim 10, **characterized in that** a curve marked by the subject on the display device using an input appliance of the computer is stored in the computer.

12. A computer with a display device and an input appliance, set up to execute the computer program as claimed in claim 9 or 11.

13. The computer as claimed in claim 12, **characterized in that** the display device can be pivoted by 90 degrees with respect to the main viewing direction of the subject.

14. The computer as claimed in claim 12 or 13, **characterized in that** an illumination apparatus is arranged in the region of the display device.

## Revendications

1. Procédé pour l'exécution d'un test visuel de contraste chez un sujet pour la détermination de la fonction de sensibilité aux contrastes, dans lequel on donne à observer au sujet une représentation d'un réseau sinusoïdal continu (1) dans lequel la fréquence locale varie continuellement selon la direction d'un premier axe (X) et dans lequel le contraste varie continuellement selon la direction d'un second axe (Y) perpendiculaire au premier axe (X), la fonction de sensibilité au contraste étant définie par une courbe (2) qui entoure une région (3) du réseau sinusoïdal (1) dans laquelle le sujet perçoit le dessin du réseau sinusoïdal lors de l'observation, **caractérisé en ce que** les courbes enregistrées (2', 2") sont évaluées ou comparées à l'aide d'une métrique appropriée, le visus maximal perçu, le rapport de contraste minimal perçu et la forme de la courbe de sensibilité au contraste étant pris en compte dans l'évaluation.

2. Procédé selon la revendication 1, **caractérisé en ce que** la fréquence locale du réseau sinusoïdal (1) croît continuellement en partant du second axe (Y) selon la direction du premier axe (X), et **en ce que** le contraste du réseau sinusoïdal décroît continuellement à partir du premier axe (X) selon la direction du second axe (Y).

3. Procédé selon la revendication 1 ou 2, **caractérisé en ce que** la fréquence locale et/ou les zones de contraste du réseau sinusoïdal continu (1) peuvent être adaptées.

4. Procédé selon la revendication 1, 2 ou 3, **caractérisé en ce que** le test visuel de contraste est exécuté sur un ordinateur et la représentation du réseau sinusoïdal continu (1) s'effectue sur un dispositif d'affichage de l'ordinateur.

5. Procédé selon la revendication 4, **caractérisé en ce que** la représentation du réseau sinusoïdal continu (1) sur le dispositif d'affichage de l'ordinateur peut être tournée de 90 degrés par rapport à la direction de visée principale du sujet.

6. Procédé selon l'une des revendications 1 à 5, **caractérisé en ce que** le test de contraste est exécuté plusieurs fois, en particulier dans des conditions d'environnement prédéterminées, les courbes de sensibilité au contraste étant enregistrées.

7. Procédé selon l'une des revendications 1 à 6, **caractérisé en ce que** le test visuel de contraste est exécuté dans des conditions d'éblouissement.

8. Procédé selon l'une des revendications 4 à 7, **caractérisé en ce que** le sujet trace lui-même la courbe ou la fonction de sensibilité au contraste sur le dispositif d'affichage au moyen d'un dispositif d'entrée de l'ordinateur, la courbe tracée (2) étant mémorisée dans l'ordinateur.

9. Programme d'ordinateur comportant des moyens de codage de programme pour exécuter toutes les étapes d'une quelconque des revendications 1 à 7 lorsque le programme est exécuté sur un ordinateur.

10. Programme d'ordinateur comportant des moyens de codage de programme qui sont enregistrés sur un support de données pouvant être lu par un ordinateur pour exécuter toutes les étapes d'une quelconque des revendications 1 à 7, lorsque le programme est exécuté sur un ordinateur.

11. Programme d'ordinateur selon la revendication 9 ou produit programme d'ordinateur selon la revendication 10, **caractérisé en ce qu'**une courbe tracée sur le dispositif d'affichage par le sujet au moyen d'un dispositif d'entrée de l'ordinateur est mémorisée dans l'ordinateur.

12. Ordinateur comportant un dispositif d'affichage et un dispositif d'entrée et conçu pour l'exécution du programme d'ordinateur selon la revendication 9 ou 11.

13. Ordinateur selon la revendication 12, **caractérisé en ce que** le dispositif d'affichage peut être tourné de 90 degrés par rapport à la direction de visée principale du sujet.

14. Ordinateur selon la revendication 12 ou 13, **caractérisé en ce qu'**un dispositif d'éclairage est agencé dans la région du dispositif d'affichage
